# EUROPEAN PATENT SPECIFICATION

(11) **EP 1 309 583 B1**
(45) Date of publication and mention of the grant of the patent: **21.06.2006**
(21) Application number: 01974118.0
(22) Date of filing: 06.08.2001
(51) Int. Cl.: C07D 309/30, C07C 271/22, C07F 7/18

(54) **PROCESS FOR PREPARING DISCODERMOLIDE AND ANALOGUES THEREOF**
VERFAHREN ZU HERSTELLUNG VON DISCODERMOLIDEN UND ÄHNLICHEN VERBINDUNGEN
PROCEDE DE PREPARATION DE DISCODERMOLIDE ET DE SES ANALOGUES

(30) Priority: 07.08.2000 US 633753
(43) Date of publication of application: 14.05.2003
(73) Proprietor: Novartis AG, 4002 Basel (CH); Novartis Pharma GmbH, 1230 Wien (AT)
(72) Inventor: KINDER, Frederick, Ray, Morristown, NJ 07960 (US)
(74) Representative: Gros, Florent
(86) International application number: PCT/EP2001/009068
(87) International publication number: WO 2002/012220

(56) References cited:
- WO-A-91/01982

## Description

The invention relates to a process for preparing discodermolide and analogues thereof, to novel compounds utilized in the process and to novel compounds prepared by the process.

### BACKGROUND OF THE INVENTION

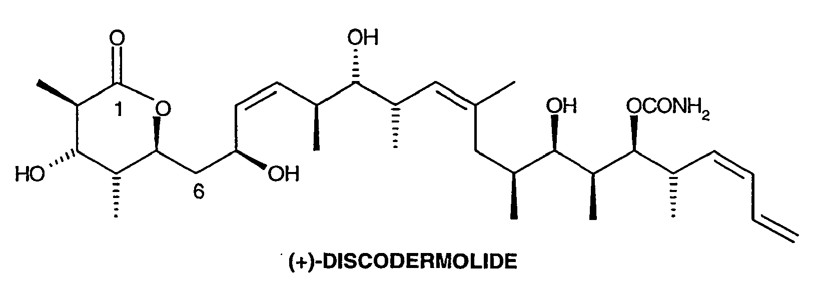

(+)-Discodermolide is a novel polyketide natural product that was isolated from extracts of the marine sponge *Discodermolide dissoluta* by researchers at the Harbor Branch Oceanographic Institution (HBOI) (Gunasekera SP, Gunasekera M, Longley RE, Schulte GK. Discodermolide: a new bioactive polyhydroxylated lactone from the marine sponge *Discodermia dissoluta.* [published erratum appears in J. Org. Chem. 1991;56:1346]. J. Org. Chem. 1990;55:4912-15.). Discodermolide lacks obvious structural resemblance to paclitaxel, yet it shares with paclitaxel (the active substance in the drug Taxol) the ability to stabilize microtubules. In mechanism-based assays, discodermolide is more effective than paclitaxel. In fact, of the handful of compounds known to induce polymerization of purified tubulin, discodermolide is the most potent. However, microtubules, a major structural component in cells, are not simple equlibrium polymers of tubulin. They exist as regulated GTP-driven dynamic assemblies of heterodimers of α and β tubulin. Although the dynamics are relatively slow in interphase cells, upon entering mitosis, the rate of growing and shortening increases 20 to 100-fold - the average microtubule turns over half the tubulin subunits every ten seconds. This change in rate allows the cytoskeletal microtubule network to dismantle and a bipolar spindle-shaped array of microtubules to assemble. The spindle attaches to chromosomes and moves them apart. The response to complete suppression of microtubule dynamics in cells is death. However, mitotic cells are more sensitive and the tolerance threshold appears to be cell-type specific. Molecules like paclitaxel that bind with high affinity to microtubules disrupt the dynamics process in tumor cells with lethal results even when the ratio of bound drug to tubulin is very low. Discodermolide binds to tubulin competitively with paclitaxel. Since paclitaxel has proven to be useful in treating some cancers, other compounds of the same mechanistic class may have utility against hyperproliferative disorders.

Future development of discodermolide or structurally related analogues is hindered by the lack of a reliable natural source of the compound or a feasible synthetic route. Naturally occurring discodermolide is scarce and harvesting the producing organism presents logistical problems. Accordingly, there is an ever-growing need for improved syntheses which enable the preparation of commercially acceptable quantities of discodermolide and structurally related analogues.

### SUMMARY OF THE INVENTION

The present invention relates to a more practical synthesis of discodermolide and analogues thereof. In another embodiment, the instant invention relates to novel compounds useful in the preparation of discodermolide and analogues thereof. In a further embodiment, the instant invention relates to novel compounds which are prepared by the process of the instant invention.

### DETAILED DESCRIPTION OF THE INVENTION

The essence of the instant invention is the discovery of a more practical synthesis for discodermolide and analogues thereof. More particularly, it has been discovered that discodermolide and analogues thereof can be prepared by a three-step reaction as follows: 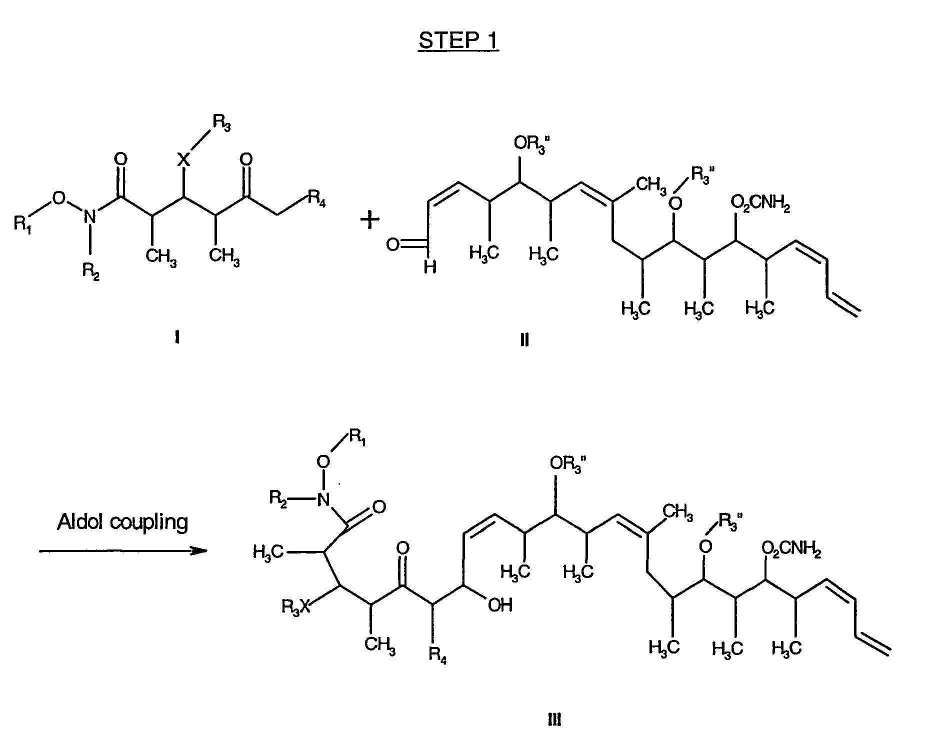 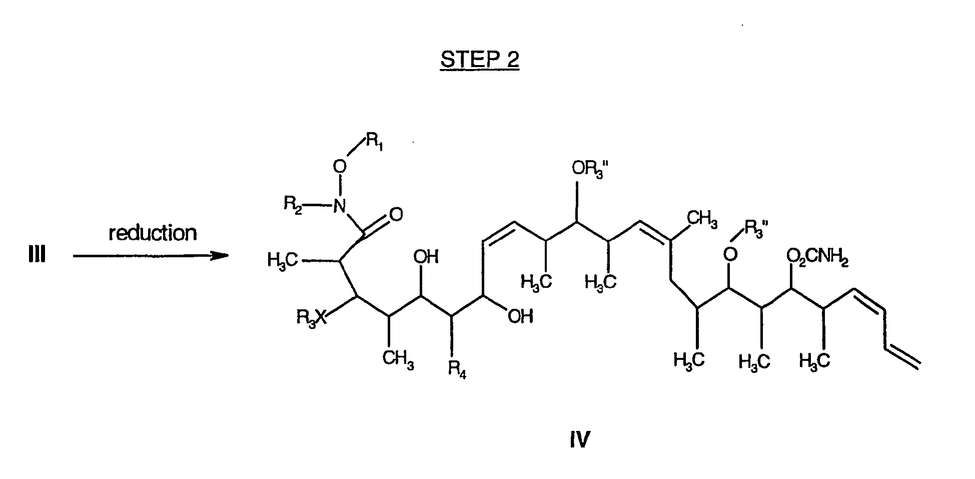 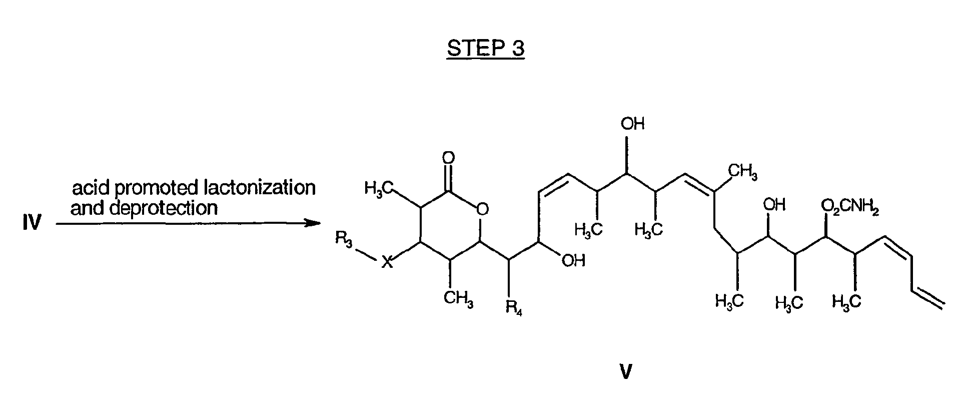 where R₁ is (C₁₋₆) alkyl, benzyl or an acid labile hydroxyl protecting group; R₂ is (C₁₋₆) alkyl or benzyl; R₃ is hydrogen, (C₁₋₆) alkyl, benzyl, C(O)(C₁₋₁₂) alkyl, C(O)Ph, C(O)O(C₁₋₁₂) alkyl, C(O)OPh, C(O)NH(C₁₋₁₂) alkyl, C(O)NHPh or an acid labile hydroxyl protecting group; R₃" is an acid labile hydroxyl protecting group; R₄ is hydrogen or methyl; and X is O, NH, NCH₃, S or CH₂, with the proviso that when X is O and R₃ is an acid labile hydroxyl protecting group in the compound of formula I, the "-X-R₃" moiety in the compound of formula V is -OH.

As to the individual steps, Step 1 involves the coupling of a ketone compound of formula I with an aldehyde compound of formula II via an aldol reaction to obtain a β-hydroxyketone compound of formula III. The coupling is conveniently carried out with between 1 and 20, preferably between 5 and 15, equivalents of the ketone compound of formula I relative to the aldehyde compound of formula II. The coupling is conducted in the presence of: 1) a dialkylboron halide or triflate, preferably a chiral boron chloride or triflate, more preferably β-chlorodiisopinocamphenylborane; 2) a base, preferably an amine, more preferably triethylamine; and 3) a polar organic solvent, preferably an ether, more preferably diethyl ether, at a temperature of between - 100ºC and 20ºC, preferably between -78ºC and -20ºC, for a period of between 2 and 72 hours, preferably for 16 hours.

Step 2 concerns the reduction of the β-hydroxyketone compound of formula III and, more particularly, the ketone group common to such compounds, to obtain a 1,3-diol compound of formula IV. The reduction is conducted in the presence of: 1) a ketone reducing agent, preferably a borohydride such as tetramethylammonium triacetoxyborohydride; 2) a polar organic solvent, preferably acetonitrile; and 3) a protic solvent, preferably a carboxylic acid, such as acetic acid, at a temperature of between 78ºC and 20ºC, preferably between -40ºC and -10ºC, for a period of between 2 and 72 hours, preferably for 16 hours.

As to Step 3, it involves the lactonization and deprotection of the acid labile hydroxyl protecting groups of a compound of formula IV to obtain a compound of formula V. The lactonization and deprotection reaction is conducted in the presence of: 1) a protic acid, preferably an aqueous protic acid solution, preferably an aqueous hydrogen halide solution, such as aqueous hydrogen chloride; and 2) a polar organic solvent, preferably a mixture of polar organic solvents, more preferably a mixture of an aliphatic alcohol and an ether, such as methanol and tetrahydrofuran, at a temperature of between -20ºC and 40ºC, preferably between 20ºC and 25ºC, for a period of 8 hours and 7 days, preferably between 16 and 72 hours, more preferably between 24 and 48 hours.

In another embodiment, the instant invention relates to the novel ketone compounds of formula I: 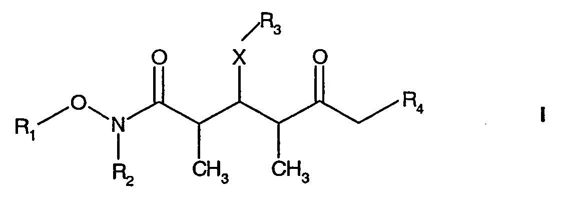
where R₁ is (C₁₋₆) alkyl, benzyl, or an acid labile hydroxyl protecting group;
R₂ is (C₁₋₆) alkyl, or benzyl;
R₃ is hydrogen, (C₁₋₆) alkyl, benzyl, C(O)(C₁₋₁₂) alkyl, C(O)Ph, C(O)O(C₁₋₁₂)alkyl, C(O)OPh, C(O)NH(C₁₋₁₂)alkyl, C(O)NHPh, or an acid labile hydroxyl protecting group;
R₄ is hydrogen or methyl; and
X is O, NH, NCH₃, S or CH₂.

Preferred compounds are those of formula la: 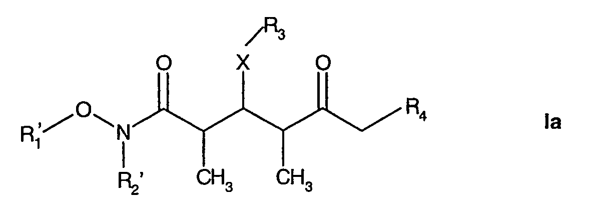
where each of R₁' and R₂' is (C₁₋₆) alkyl;
X is O, or CH₂; and
R₃ and R₄ are as defined above.

More preferred compounds are those of formula Ib: 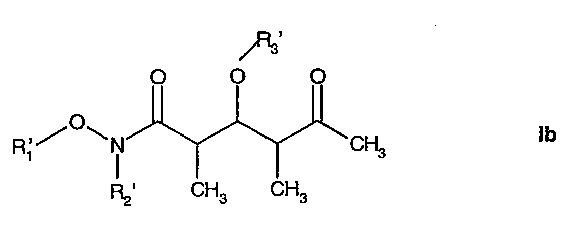
where R₃' is (C₁₋₆)alkyl, C(O)(C₁₋₁₂)alkyl, benzyl, C(O)O(C₁₋₁₂)alkyl, or an acid labile hydroxyl protecting group; and
R₁' and R₂' are as defined above.

Even more preferred compound are those of formula Ic: 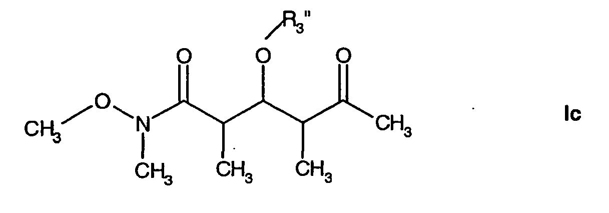 where R₃" is an acid labile hydroxyl protecting group.

In the above definitions: the term "(C₁₋₆) alkyl" as used herein refers to a straight or branched chain group consisting solely of carbon and hydrogen and having from 1 to 6 carbon atoms, whereas the term "(C₁₋₁₂) alkyl" as used herein refers to a straight or branched chain group consisting solely of carbon and hydrogen and having from 1 to 12 carbon atoms. Examples of "alkyl" groups include methyl, ethyl, propyl, butyl, pentyl, 3-methylpentyl, etc.

The term "acid labile hydroxyl protecting groups" as used herein refers to any oxygen bound group that can be removed upon exposure to an acid. Numerous examples of these groups are known by those skilled in the art and can be found in Greene and Wuts, Protective Groups in Organic Synthesis, 2d edition, John Wiley & Sons, New York, 1991. Specific examples include, but are not limited to, *t*-butyldimethylsilyl, triethylsilyl, *t-*butyldiphenylsilyl, triisopropylsilyl, methoxymethyl, and tetrahydropyranyl.

In a further embodiment, the instant invention relates to a process for preparing the novel compounds of formula I. More particularly, the compounds of formula I may be prepared as depicted below: 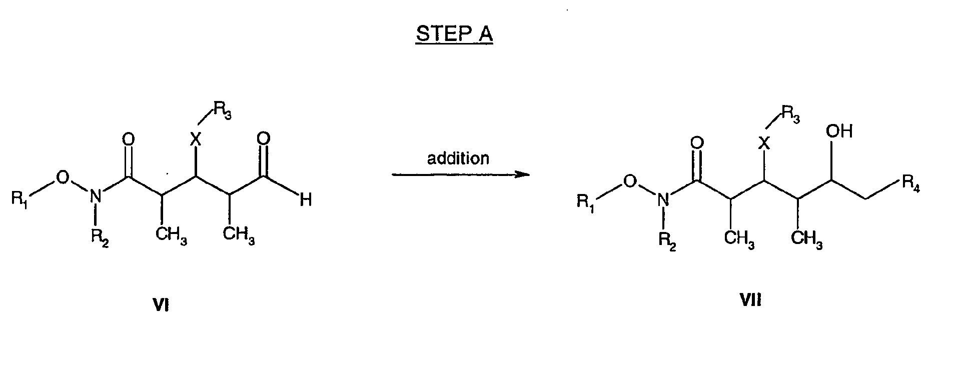 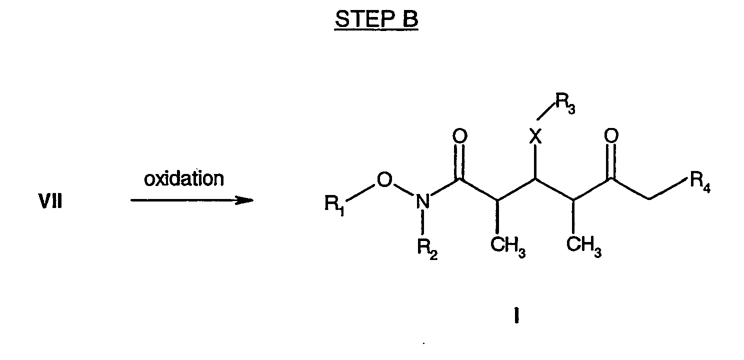 where R₁, R₂, R₃, R₄ and X are as defined above.

As to the individual steps, Step A involves the addition of a methyl or ethyl group to an aldehyde compound of formula VI to obtain an alcohol compound of formula VII. The addition is carried out in the presence of: 1) an organometallic reagent, preferably an alkyllithium or alkylmagnesium halide such as methylmagnesium bromide; and 2) a polar organic solvent, preferably an ether such as diethyl ether, at a temperature of between - 78ºC and 40ºC, preferably at -78ºC and 0ºC., more preferably at about -40ºC., for a period of between 5 minutes and 24 hours, preferably between 30 minutes and 2 hours, more preferably for about 1 hour.

Step B involves the oxidation of an alcohol compound of formula VII to obtain the desired ketone compound of formula I. The oxidation is carried out in the presence of: 1) an oxidant, preferably a combination of dimethylsulfoxide and an activating agent, more preferably a combination of dimethylsulfoxide and sulfur trioxide complex with pyridine; 2) a base, preferably an organic base, more preferably a trialkylamine such as triethylamine; and 3) a polar organic solvent, preferably a chlorinated hydrocarbon such as dichloromethane. The oxidation is suitably carried out a temperature of between -78ºC and 40ºC, preferably between 5ºC and 20ºC, for a period of between 5 minutes and 24 hours, preferably between 1 hour and 12 hours, more preferably between 4 and 6 hours.

The aldehyde compounds of formulae II and VI are either known or may be prepared analogous to processes set forth in the literature for other structurally similar aldehydes.

In still another embodiment, the instant invention relates to the novel β-hydroxyketone compounds of formula III and the novel 1,3-diol compounds of formula IV.

Although the product of each reaction described above may, if desired, be purified by conventional techniques such as chromatography or recrystallization (if a solid), the crude product of one reaction is advantageously employed in the following reaction without purification.

As is evident to those skilled in the art, compounds of formulae I and III-V contain asymmetric carbon atoms and, therefore, it should be understood that the individual stereoisomers are contemplated as being included within the scope of this invention.

The following examples are for purposes of illustration only and are not intended to limit in any way the scope of the instant invention.

### EXAMPLE 1

### (2R,3S,4R)-3-[[(1,1-dimethylethyl)dimethylsilyl]oxy]-N-methoxy-N,2,4-trimethy1-5-oxo-hexanamide

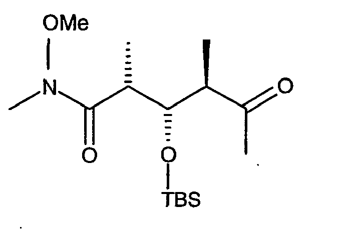

To a solution of 13.1 g (39.5 mmol) of the diastereomeric mixture prepared in Stage 1.1 in a mixture comprising 150 mL of methylene chloride, 50 mL of dimethylsulfoxide and 25 mL of triethylamine is added, dropwise via an addition funnel, 19.0 g (120 mmol) of sulfur trioxide pyridine complex in 150 mL of dimethylsulfoxide at 0ºC. The resultant solution is then stirred for 1 ½ hours at 0ºC, after which time the reaction mixture is concentrated via a rotary evaporator in a cooling both at <10ºC. The solution is then diluted with 200 mL of ether, and then extracted successively with 200 mL of a 1 M sodium hydrogen sulfate solution and 200 mL of brine. The organic layer is then dried over sodium sulfate and the crude product mixture is purified by flash chromatography employing hexane as the eluent initially and then an eluent mixture of 5% ethyl acetate in hexane to yield the desired compound as a clear oil.

¹H NMR (300 MHz, CDCl₃) δ 4.23 (dd, J = 7.5, 4.2 Hz, 2H), 3.61 (s, 3H), 3.01 (s, 3H), 2.92 (m, 2H), 2.64 (m, 2H), 2.08 (s, 3H), 1.03 (d, J = 6.8 Hz, 3H), 0.98 (d, J = 7.2 Hz, 3H), 0.81 (s, 9H), 0.00 (s, 6H).

### Stage 1.1: (2R,3S,4S)-3-[[(1,1-dimethylethyl)dimethylsilyl]oxy]-5-hydroxy-N-methoxy-N,2,4-trimethyl-hexanamide

To a solution of 6.9 g (21.8 mmol) of (2*R*,3*S*,4*R*)-3-[[(1,1-dimethylethyl)dimethylsilyl]oxy]-*N*-methoxy-*N*,2,4-trimethyl-5-oxo-pentanamide in 50 mL of ether is added 10.2 mL (30.5 mmol) of a 3M solution of methylmagnesium bromide in ether, dropwise, at -40ºC, after which the mixture is stirred at -20ºC for 1 hour. The reaction mixture is then dilluted with 200 mL of ether and the reaction is quenched by adding the reaction mixture to 20 g of crushed ice at 0ºC. The mixture is then washed with 100 mL of 1 M sodium hydrogen sulfate solution and partitioned with two 150 mL portions of ether. The organic layers are then combined and dried over sodium sulfate. The crude product mixture is then chromatographed employing an eluent mixture of 20 % ethyl acetate in hexane initially and then an eluent mixture of 40% ethyl acetate in hexane to yield two diastereomers as light yellow oils which are used in the next step without further purification.

Diastereomer 1: ¹H NMR( 300 MHz, CDCl₃): δ 4.08 (dd, J = 9.8, 6.8 Hz, 2H), 3.64 (s, 3H), 3.54 (d, J = 2.6Hz, 2H), 3.08 (s, 3H), 3.00 (m, 2H), 1.48 (m, 2H), 1.07 (d, J = 6.8 Hz, 3H), 1.05 (d, J = 6.4 Hz, 3H), 0.81 (s, 9H), 0.76 (d, J = 7.2Hz, 2H), 0.01 (d, J = 3.8 Hz, 6H). Diastereomer 2: ¹H NMR( 300 MHz, CDCl₃): δ 4.19 (dd, J = 12.1, 6.0 Hz, 2H), 3.86 (dd, J = 9.0, 1.51 Hz, 2H), 3.56 (s, 3H), 3.18 (m, 2H), 3.01 (s, 3H), 1.28 (m, 2H), 1.06 (d, J = 6.8 Hz, 3H), 0.94 (d, 6.4 Hz, 3H), 0.88 (d, 7.2 Hz, 3H), 0.78 (s, 9H), 0.01 (d, 1.3Hz, 6H).

### EXAMPLE 2

(2*R*,3*S*,4*R*,7*S*,8*Z*,10*S*,11*S*,12*S*,13*Z*,16*S*,17*R*,18*R*,19*S*,20*S*,21*E*)-19-[(aminocarbonyl)oxyl]-3,11,17-*tris*[[(1,1-dimethylethyl)dimethylsilyl]oxy]-7-hydroxy-*N*-methoxy-*N*,2,4,10,12,14, 16,18,20-nonamethyl-5-oxo-8,13,21,23-tetracosatetraenamide

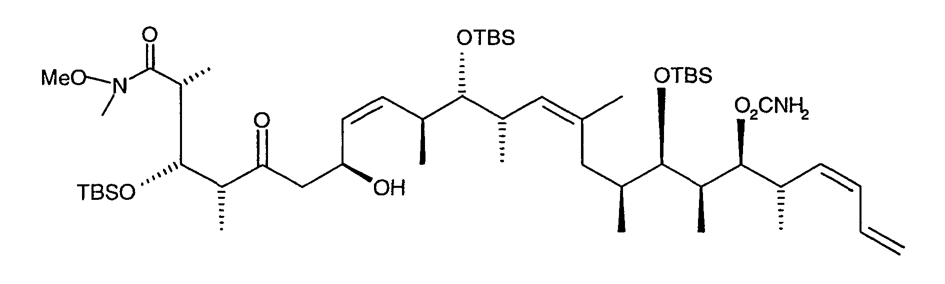

To a stirred solution of 2.18 g (6.79 mmol, 10 equiv.) of (+)-β-chlorodiisopinocamphenylborane in 4 mL of diethyl ether at 0ºC is added 1.04 mL (11 equiv., distilled over calcium hydride) of triethylamine and then a solution of 2.25 g (6.79 mmol, 10 equiv.) of the compound of Example 1 in 3 mL of diethylether. After stirring for 2 hours at 0ºC, the mixture is cooled to -78ºC, after which time a pre-cooled (-78ºC) solution of 450 mg (0.679 mmol) of (2*Z*,4*S*,5*S*,6*S*,7*Z*,10*S*, 11*R*,12*R*,13*S*,14*S*,15*E*)-13-[(amino-carbonyl)oxyl]-5,11-*bis*[[(1,1-dimethylethyl)dimethylsilyl]oxy]-4,6,8,10,12,14-hexamethyl-2,7,15,17-octadecatetraenal in 4mL of diethylether is added via cannula. After the resultant mixture is maintained at a temperature of -78ºC for 3 hours, it is transferred to a freezer (-27ºC) for 16 hours. The reaction is then quenched with 8 mL of methanol (the pH of which is adjusted to 7 with 12 mL of buffer solution) and 4 mL of 50% hydrogen peroxide solution at 0ºC. After stirring for 2 hours at 25ºC, the organic layers are separated. The aqueous layer is then extracted five times with 25 mL portions of dichloromethane. The combined organic layers are then dried over magnesium sulfate, concentrated using a rotary evaporator and chromatographed (Biotage, silica gel, gradient 10-30% ethyl acetate/hexane) to yield the desired compound as a colorless, highly viscous oil.

[α]_{D}+12.56° (c= 1.0, CH₂Cl₂); IR (CH₂Cl₂) 3547 (m, OH), 3359 (m, CONH2), 2958 (vs), 2990 (vs), 1729 (vs, C=O), 1664 (m), 1462 (s), 1385 (s), 1254(s), 1037 (s), 1037 (s), 1004 (s), 835 (vs); ¹H NMR (500MHz, CDCl₃) δ 6.61 (1H, ddd, J= 17.1, 10.5, 10.5Hz, H₂₃), 6.03 (1 H, dd, J = 11.0, 11.0 Hz, H₂₂), 5.50 (1 H, dd,*J*= 10.6, 10.6 Hz, H₉), 5.37 (1 H, dd, *J*=10.6, 10.5Hz, H₂₁), 5.35 (1 H, dd, *J=* 10.8, 8.5 Hz, H₈), 5.23 (1 H, dd, *J =* 15.3, 2.1 Hz, H_{24A}), 5.13 (1 H, d, J= 10.2 Hz, H_{24B}), 5.05 (1 H, d, J = 10.0 Hz, H₁₃), 4.79 (1 H, t, *J=* 8.0 Hz, H₇), 4.72 (1H, t, J= 5.9 Hz, H₁₉), 4.60-4.50 (2H, br, CONH₂), 4.33 (1 H, dd, J= 6.9, 4.3 Hz, H₃), 3.74 (3H, s, NOCH₃), 3.43 (1 H, dd, J = 5.0, 4.1 Hz, H₁₇), 3.31 (1 H, dd, J = 5.2, 5.1 Hz, H₁₁), 3.13 (3H, s, NCH₃), 3.08 (1 H, br, OH), 3.00 (1 H, m, H₂₀), 2.78-2.69 (2H, m, H4 +H_{6A}), 2.70-2.62 (1 H, m, H₁₀), 2.66-2.54 (2H, m, H₂ + H_{6B}), 2.49-2.45 (1 H, m, H₁₂), 2.12(1 H, dd, J = 12.4, 12.3 Hz, H_{15A}), 1.93-1.86 (2H, m, H₁₆+H₁₈), 1.76-1.65 (1 H, m, H15B), 1.62 (3H, s, Me₁₄), 1.14 (3H, d, J = 7.0 Hz, Me₂), 1.11 (3H, d, J = 7.0 Hz, Me₄), 1.00 (3H, d, J = 3.1 Hz, Me₂₀), 0.99 (3H, d, J = 3.3 Hz, Me₁₀), 0.96-0.90 (21 H, m, Me₁₈ + 2xSiC(CH₃)₃), 0.88 (3H, d, J = 6.6 Hz,Me₁₂), 0.83 (9H, s, SiC(CH₃)₃), 0.73 (3H, d, J= 6.7 Hz,Me₁₆), 0.10 & 0.08 & 0.04 & 0.03 & 0.03 & 0.01 (6x3H, 3xSi(CH₃)₂);
¹³C NMR (100.6MHz, CDCl₃) δ 212.9, 175.9, 156.9, 136.0, 133.7, 132.1, 131.9, 131.3, 129.8, 129.6, 117.9, 80.6, 78.7, 76.8, 73.6, 64.9, 62.1, 61.3, 54.7, 53.1, 51.7, 49.0, 45.1, 44.9, 37.9, 37.1, 36.2, 35.9, 35.0, 34.4, 30.0, 29.1, 26.26, 26.24, 25.97, 23.0, 18.51, 18.5, 18.43, 18.14, 17.43, 16.44, 13.5, 10.99, 10.1, -3.29, -3.4, -3.5, -3.9, -4.1, -4.4; m/z (ESI+) 1017 (100 (MNa⁺)).

### EXAMPLE 3

### (2R,3S,4S,5S,7S,8Z,10S,11S,12S,13Z,16S,17R,18R,19S,20S,21E)-19-[(aminocarbonyl)-oxyl]-3,11,17-tris[[(1,1-dimethylethyl)dimethylsilyl]oxy]-5,7-dihydroxy-N-methoxy-N,2,4,10,12,14,16,18,20-nonamethyl-8,13,21,23-tetracosatetraenamide

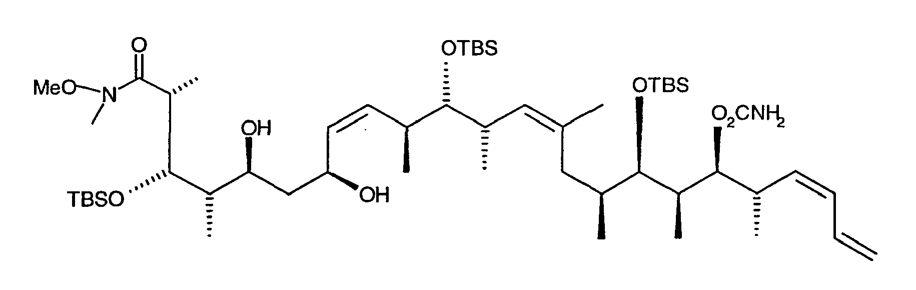

To a solution of 1.02 g (3.9 mmol) of tetramethylammonium triacetoxyborohydride in 2.2 mL of anhydrous acetonitrile is added 2.2 mL of anhydrous acetic acid and the mixture is stirred at ambient temperature for 30 min. The mixture is then cooled to -29ºC and to the cooled mixture is added a solution of 450 mg (0.453 mmol) of the compound of Example 2 in 1 mL of anhydrous acetonitrile. The resultant mixture is then stirred at -29°C for 18 hours, after which time the reaction is quenched with 2 mL of 0.5N aqueous sodium potassium tartrate. The mixture is then allowed to warm slowly to ambient temperature, after which it is diluted with methylene chloride and washed with saturated sodium bicarbonate. The aqueous layer is then extracted four times with methylene chloride. The combined organic layers are then washed with brine, dried with sodium sulfate and concentrated *in vacuo.* The resultant residue is then purified by flash chromatography (Biotage, silica gel, gradient 10-30% ethyl acetate/hexane) to yield the desired compound as a white solid.

[α]+29.75 degree (*c*= 0.87, CH₂Cl₂); ¹H NMR (499.87MHz, CDCl₃) δ 6.60 (1H, ddd, *J*= 16.8, 10.5, 10.5Hz, H₂₃), 6.02 (1 H, t, *J*= 11.0, H₂₂), 5.48 (1 H, dd, *J*= 10.0, 9.8 Hz, Hg), 5.37 (1 H, dd, *J=* 10.6, 11.2Hz, H₂₁), 5.35 (1 H, dd, *J=* 10.8, 8.5 Hz, H₈), 5.22 (1 H, d, *J =* 15.8 Hz, H_{24A}), 5.12 (1 H, d, *J =* 10.2 Hz, H_{24B}), 4.98 (1 H, d, *J* =10.1 Hz, H₁₃), 4.79 (1 H, t, *J=* 6.3 Hz, H₇), 4.65 (1 H, t, *J =* 5.9 Hz, H₁₉), 4.60-4.50 (2H, br, CONH₂), 4.20 (1 H, dd, *J =* 7.7, 2.3 Hz, H₃), 3.92, (1 H, m, H₅), 3.73 (3H, s, NOCH₃), 3.45 (1 H, br, OH-5), 3.41 (1H, dd, *J=* 10.9, 4.7 Hz, H₁₇), 3.31 (1 H, dd, *J =* 5.2, 5.1 Hz, H₁₁), 3.18 (3H, s, NCH₃), 3.08 (1 H, br, OH), 2.99 (1 H, m, H₂₀), 267 (1 H, m, H₁₀), 2.43-2.41 (2H, m), 2.11 (1 H, t, *J =* 12.3 Hz), 1.90-1.87 (2H, m), 1.76-1.58 (10H, m), 1.25 (3H, t, Me), 1.17 (3H, d, *J =* 7.1 Hz, Me), 0.99 (3H, d, *J*= 6.4 Hz, Me), 0.97 (3H, d, *J =* 6.5 Hz, Me), 0.93-0.83 (30H, m, Me + 3xSiC(CH₃)₃), 0.71 (3H, d, *J* = 6.8 Hz,Me), 0.10 & 0.08 & 0.04 & 0.03 & 0.03 & 0.01 (6x3H, 3xSi(CH₃)₂). ¹³C NMR (100.6MHz, CDCl₃) δ 156.88, 140.02, 134.19, 133.66, 132.10, 131.88, 131.40, 131.30, 131.11, 130.06, 129.79, 117.91, 115.44, 80.79, 80.69, 78.61, 78.32, 74.31, 70.68, 65.55, 61.66, 45.69, 40.38, 38.36, 37.92, 37.83, 37.29, 36.29, 35.07, 34.91, 34.45, 32.36, 29.68, 26.21, 26.12, 26.03, 25.95, 22.95, 18.52, 18.43, 18.12, 17.41, 17.07, 16.57, 13.44, 12.29, 10.32, 10.14, -3.20, -3.43, -3.96, -4.16, -4.48. m/z (ESI+) 1019 (100 (MNa⁺)).

### EXAMPLE 4

### Preparation of (+)- discodermolide

To a solution of 450 mg (0.452 mmol) of the compound of Example 3 in 56 mL of tetrahydrofuran is added 56 mL of an aqueous solution of 4N hydrochloric acid. The resultant solution is then stirred at room temperature for 24 hours, 10 mL of methanol is then added, and this solution is then stirred for an additional 24 hours at room temperature. To the solution is then added 50 mL of ethyl acetate, followed by the addition of sodium bicarbonate at 0°C to a pH of 8. The organic solution is then washed with brine. The aqueous layer is then extracted three times with 30 mL portions of ethyl acetate, and the combined extracts are dried over sodium sulfate. Filtration and concentration followed by flash chromatography employing an eluent mixture of 50% methylene chloride in ethyl acetate initially and then 100% ethyl acetate yields (+)- discodermolide.

[α]+22.0° (c = 1.41, MeOH); mp 122 - 124 °C; ¹³C NMR (1 00.6MHz, CDCl₃) δ 176.8, 160.33, 134.17. 133.92, 133.88, 133.59, 133.28, 131.59, 131.00, 118.80, 80.66, 80.22, 78.48, 76.48, 73.66, 63.70, 44.56, 42.60, 38.79, 37.71, 36.92, 36.77, 36.69, 34.97, 34.62, 23.45, 19.73, 18.25, 18.11, 16.05, 15.84, 13.27, 9.44. m/z (ESI+) 594 (100 (M+1⁺)).

## Claims

1. A process for preparing a compound of formula V 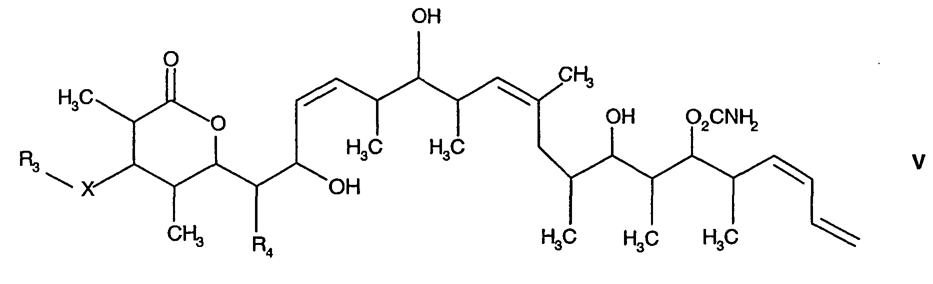 which comprises, in a first step, coupling a ketone compound of formula I 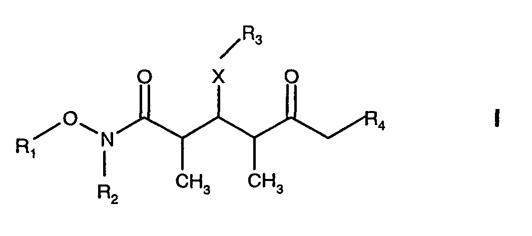 with an aldehyde compound of formula II 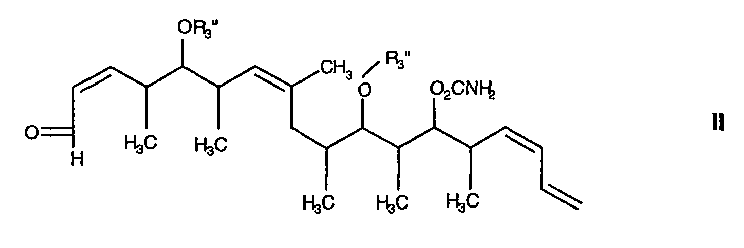 in the presence of a dialkyl boron halide or triflate, an amine base, and a polar organic solvent to obtain a β-hydroxyketone of formula III 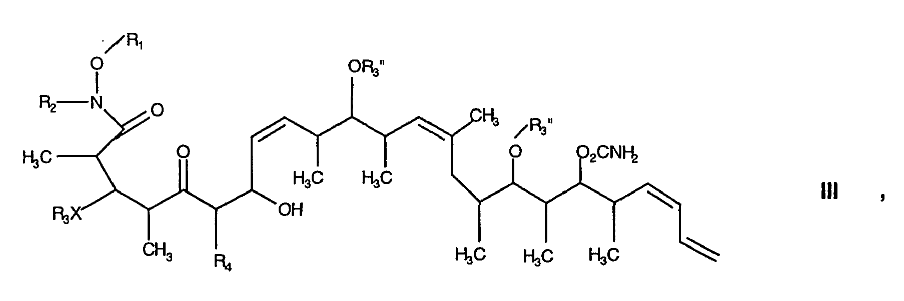 in a second step, reducing the ketone compound obtained in the first step by treating it with a borohydride reagent in a polar organic solvent and a protic solvent to obtain a 1,3-diol of formula IV 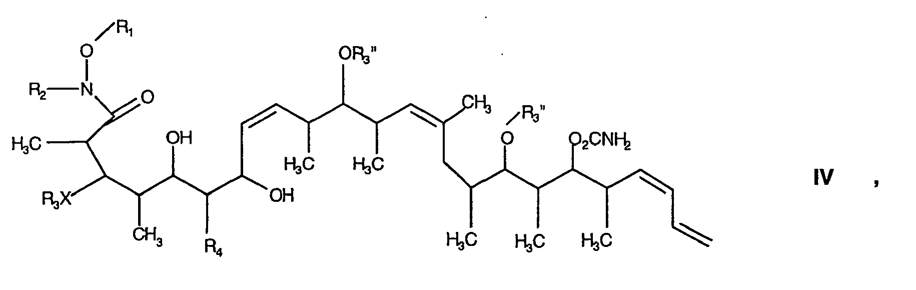 and, in a third step, lactonizing and deprotecting the acid labile hydroxyl protecting groups of the 1,3 diol obtained in the second step by treating it with a hydrogen halide dissolved in a polar solvent or mixture of solvents to obtain the desired compound of formula V, where R₁ is (C₁₋₆) alkyl, benzyl or an acid labile hydroxyl protecting group; R₂ is (C₁₋₆) alkyl or benzyl; R₃ is hydrogen, (C₁₋₆) alkyl, benzyl, C(O)(C₁₋₁₂) alkyl, C(O)Ph, C(O)O(C₁₋₁₂) alkyl, C(O)OPh, C(O)NH(C₁₋₁₂) alkyl, C(O)NHPh or an acid labile hydroxyl protecting group; R₃" is an acid labile hydroxyl protecting group; R₄ is hydrogen or methyl; and X is O, NH, NCH₃, S or CH₂, with the proviso that when X is O and R₃ is an acid labile hydroxyl protecting group in the compound of formula I, the "-X-R₃" moiety in the compound of formula V is -OH.

2. A process according to Claim 1 wherein the coupling step is carried out in the presence of β-chlorodiisopinocamphenylborane, triethylamine and diethyl ether, at a temperature of between -78°C and -20°C.

3. A process according to Claim 1 wherein the reduction step is carried out in the presence of tetramethylammonium triacetoxyborohydride, acetonitrile and acetic acid, at a temperature of between -40°C and -10°C.

4. A process according to Claim 1 wherein the lactonization and deprotection of the acid labile hydroxyl protecting groups step is carried out in the presence of aqueous hydrogen chloride, methanol and tetrahydrofuran, at a temperature of between -20°C and 40°C.

5. A compound of formula I: 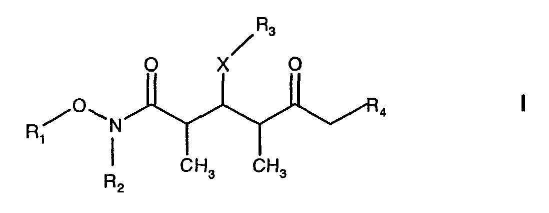
where R₁ is (C₁₋₆) alkyl, benzyl, or an acid labile hydroxyl protecting group;
R₂ is (C₁₋₆) alkyl, or benzyl;
R₃ is hydrogen, (C₁₋₆) alkyl, benzyl, C(O)(C₁₋₁₂) alkyl, C(O)Ph, C(O)O(C₁₋₁₂)alkyl, C(O)OPh, C(O)NH(C₁₋₁₂)alkyl, C(O)NHPh, or an acid labile hydroxyl protecting group;
R₄ is hydrogen or methyl; and
X is O, NH, NCH₃, S or CH₂.

6. A compound according to Claim 5 of formula la: 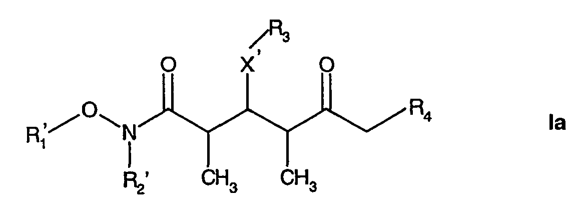
where each of R₁' and R₂' is (C₁₋₆) alkyl;
X' is O, or CH₂; and
R₃ and R₄ are as defined in Claim 5.

7. A compound according to Claim 6 of formula lb: 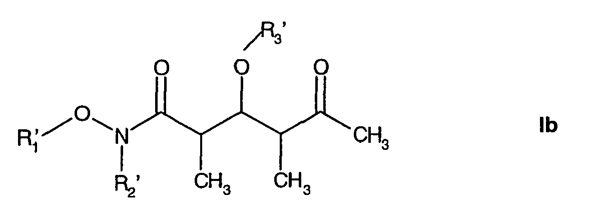
where R₃' is (C₁₋₆)alkyl, C(O)(C₁₋₁₂)alkyl, benzyl, C(O)O(C₁₋₁₂)alkyl, or an acid labile hydroxyl protecting group; and
R₁' and R₂' are as defined in Claim 6.

8. A compound according to Claim 7 of formula Ic: 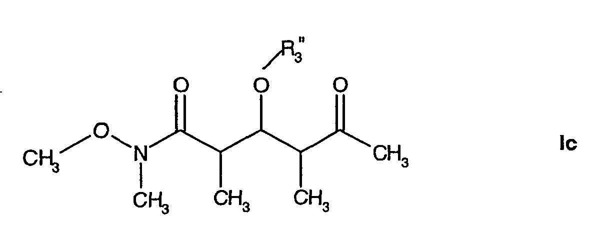 where R₃" is an acid labile hydroxyl protecting group.

9. A compound according to Claim 8 which is (2R,3S,4R)-3-[[(1,1-dimethylethyl)dimethylsilyl]oxy]-*N*-methoxy-N,2,4-trimethyl-5-oxo-hexanamide having the formula

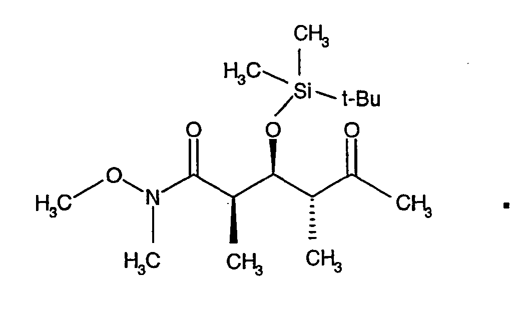

10. A process for preparing a compound of formula I according to Claim 5 which comprises, in a first step, addition of a methyl or ethyl organometallic reagent to an aldehyde of formula VI 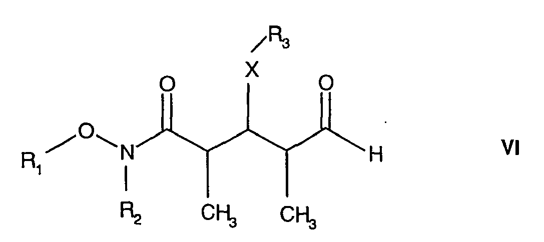 in the presence of a polar organic solvent to obtain an alcohol of formula VII 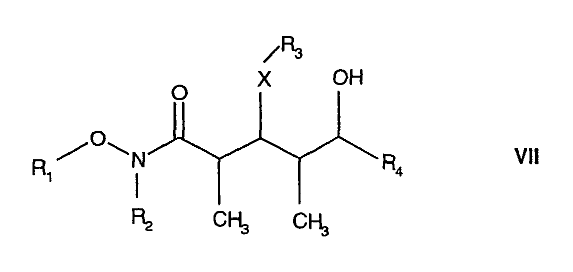 and, in a second step, oxidizing the alcohol compound prepared in the first step by treating it with an oxidant and a base in a polar organic solvent to obtain the desired compound of formula I, wherein R₁, R₂, R₃, R₄ and X are as defined in Claim 5.

11. A process according to Claim 10 wherein the addition step is carried out in the presence of methylmagnesium bromide and diethyl ether, at a temperature of between - 78ºC and 40ºC.

12. A process according to Claim 10 wherein the oxidation step is carried out in the presence of sulfur trioxide-pyridine complex, triethylamine and dichloromethane, at a temperature of between -78ºC and 40ºC.

13. A compound of formula III: 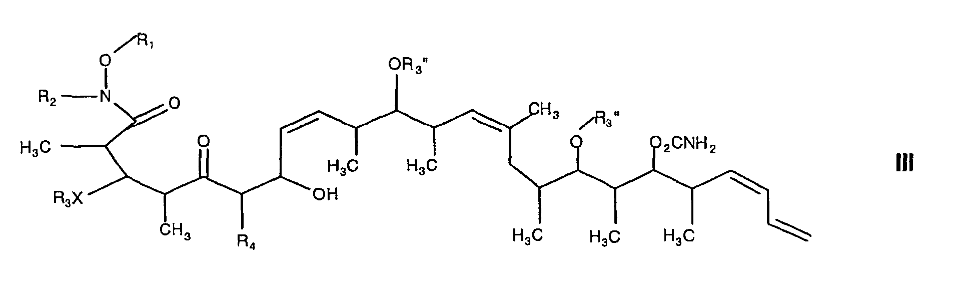
where R₁ is (C₁₋₆) alkyl, benzyl, or an acid labile hydroxyl protecting group;
R₂ is (C₁₋₆) alkyl, or benzyl;
R₃ is hydrogen, (C₁₋₆) alkyl, benzyl, C(O)(C₁₋₁₂) alkyl, C(O)Ph, C(O)O(C₁₋₁₂)alkyl, C(O)OPh, C(O)NH(C₁₋₁₂)alkyl, C(O)NHPh, or an acid labile hydroxyl protecting group;
R₄ is hydrogen or methyl;
X is O, NH, NCH₃, S or CH₂; and
each R₃" is an acid labile hydroxyl protecting group.

14. A compound according to Claim 13 which is (2*R*,3*S*,4*R*,7*S*,8*Z*,10*S*,11*S*,12*S*,13*Z*, 16*S*,17*R*,18*R*,19*S*,20*S*,21*E*)-19-[(aminocarbonyl)oxy]-3,11,17-*tris*[[(1,1-dimethylethyl)-dimethylsilyl]oxy]-7-hydroxy-*N*-methoxy-*N*,2,4,10,12,14,16,18,20-nonamethyl-5-oxo-8,13,21,23-tetracosatetraenamide having the formula

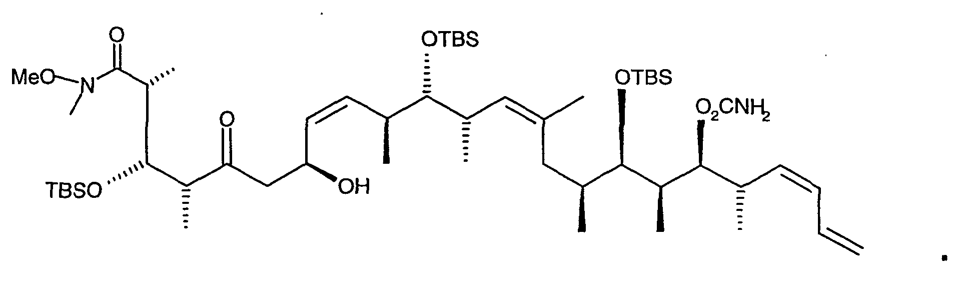

15. A compound of formula IV: 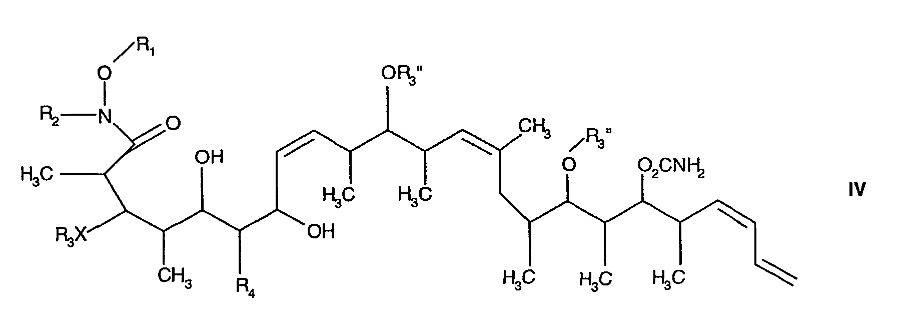
where R₁ is (C₁₋₆) alkyl, benzyl, or an acid labile hydroxyl protecting group;
R₂ is (C₁₋₆) alkyl, or benzyl;
R₃ is hydrogen, (C₁₋₆)alkyl, benzyl, C(O)(C₁₋₁₂) alkyl, C(O)Ph, C(O)O(C₁₋₁₂)alkyl, C(O)OPh, C(O)NH(C₁₋₁₂)alkyl, C(O)NHPh, or an acid labile hydroxyl protecting group;
R₄ is hydrogen or methyl;
X is O, NH, NCH₃, S or CH₂; and
each R₃" is an acid labile hydroxyl protecting group.

16. A compound according to Claim 15 which is (2*R*,3*S*,4*S*,5*S*,7*S*,8*Z*,10*S*,11*S*,12*S*, 13*Z*,16*S*,17*R*,18*R*,19*S*,20*S*,21*E*)-19-[(aminocarbonyl)oxy]-3,11,17-*tris*[[(1,1-dimethylethyl)-dimethylsilyl]oxy]-5,7-dihydroxy-*N*-methoxy-*N*,2,4,10,12,14,16,18,20-nonamethyl-8,13,21,23-tetracosatetraenamide having the formula

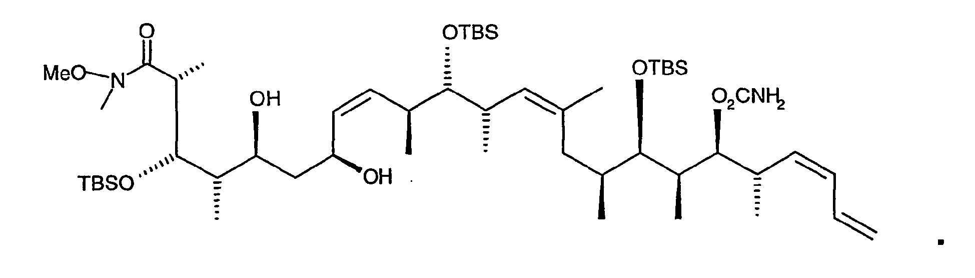

## Patentansprüche

1. Verfahren zur Herstellung einer Verbindung der Formel V 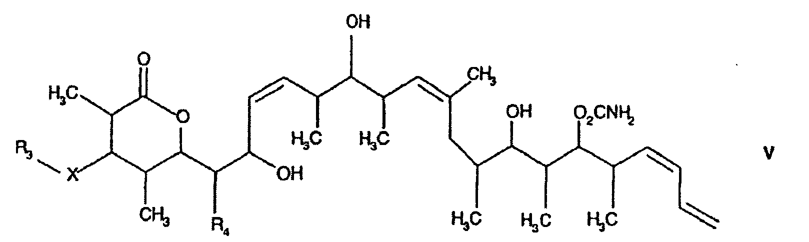 wobei das Verfahren in einer ersten Stufe eine Kupplung einer Ketonverbindung der Formel I 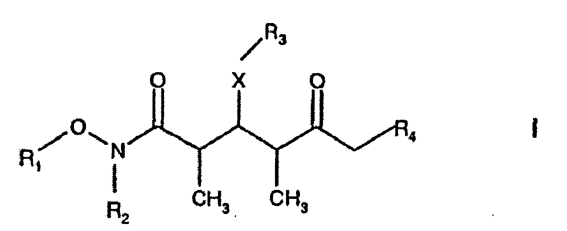 mit einer Aldehydverbindung der Formel II 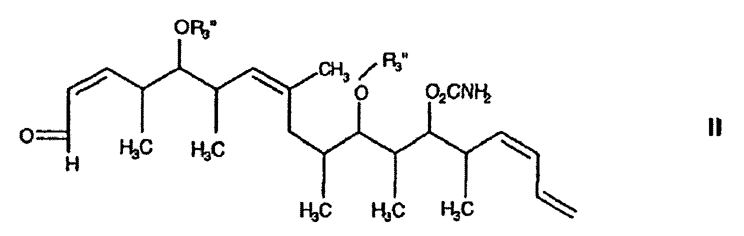 in Gegenwart eines Dialkylborhalogenids oder Triflats, einer Aminbase und eines polaren organischen Lösemittels umfasst, um ein β-Hydroxyketon der Formel III 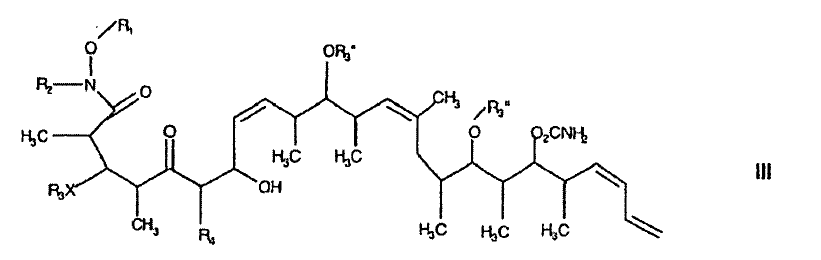 zu erhalten,
in einer zweiten Stufe eine Reduzierung der Ketonverbindung, die in der ersten Stufe erhalten wurde, durch deren Behandlung mit einem Borhydridreagenz in einem polaren organischen Lösemittel und einem protischen Lösemittel, um ein 1,3-Diol der Formel IV 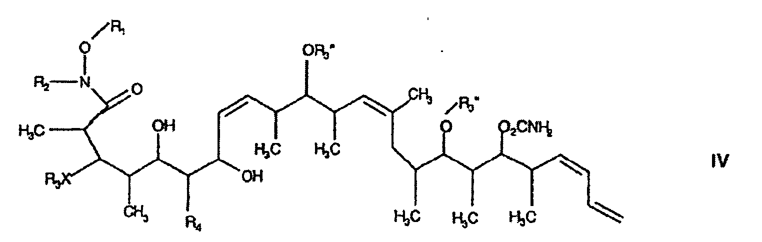 zu erhalten, und in einer dritten Stufe eine Lactonisierung und Entfernung der säurelabilen Hydroxylschutzgruppen des in der zweiten Stufe erhaltenen 1,3-Diols durch dessen Behandlung mit einem in einem polaren Lösemittel oder Gemisch von Lösemitteln gelösten Halogenwasserstoff, um die gewünschte Verbindung der Formel V zu erhalten, wobei R₁ für (C₁-C₆)-Alkyl, Benzyl oder eine säurelabile Hydroxylschutzgruppe steht; R₂ für (C₁-C₆)-Alkyl oder Benzyl steht; R₃ für Wasserstoff, (C₁-C₆)-Alkyl, Benzyl, C(O)(C₁-C₁₂)-Alkyl, C(O)Ph, C(O)O(C₁-C₁₂)-Alkyl, C(O)OPh, C(O)NH(C₁-C₁₂)-Alkyl, C(O)NHPh oder eine säurelabile Hydroxylschutzgruppe steht; R₃" für eine säurelabile Hydroxylschutzgruppe steht; R₄ für Wasserstoff oder Methyl steht; und X für O, NH, NCH₃, S oder CH₂ steht, mit der Maßgabe, dass der "-X-R₃"-Rest in der Verbindung der Formel V für -OH steht, wenn X für O steht und R₃ für eine säurelabile Hydroxylschutzgruppe in der Verbindung der Formel I steht.

2. Verfahren nach Anspruch 1, wobei die Kupplungsstufe in Gegenwart von β-Chlordiisopinocamphenylboran, Triethylamin und Diethylether bei einer Temperatur zwischen -78°C und -20°C durchgeführt wird.

3. Verfahren nach Anspruch 1, wobei die Reduktionsstufe in Gegenwart von Tetramethylammoniumtriacetoxyborhydrid, Acetonitril und Essigsäure bei einer Temperatur von zwischen -40°C und -10°C durchgeführt wird.

4. Verfahren nach Anspruch 1, wobei die Stufe der Lactonisierung und Entfernung der säurelabilen Hydroxylschutzgruppen in Gegenwart von wässrigem Chlorwasserstoff, Methanol und Tetrahydrofuran bei einer Temperatur von zwischen -20°C und 40°C ausgeführt wird.

5. Verbindung der Formel I: 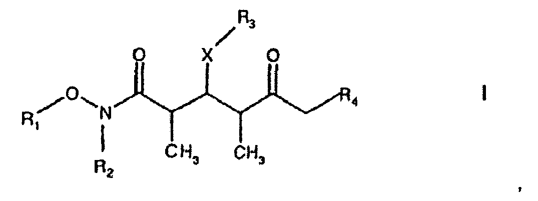 wobei
R₁ für (C₁-C₆)-Alkyl, Benzyl oder eine säurelabile Hydroxylschutzgruppe steht;
R₂ für (C₁-C₆)-Alkyl oder Benzyl steht;
R₃ für Wasserstoff, (C₁-C₆)-Alkyl, Benzyl, C(O)(C₁-C₁₂)-Alkyl, C(O)Ph, C(O)O(C₁-C₁₂)-Alkyl, C(O)OPh, C(O)NH(C₁-C₁₂)-Alkyl, C(O)NHPh oder eine säurelabile Hydroxylschutzgruppe steht;
R₄ für Wasserstoff oder Methyl steht; und
X für O, NH, NCH₃, S oder CH₂ steht.

6. Verbindung nach Anspruch 5 der Formel Ia: 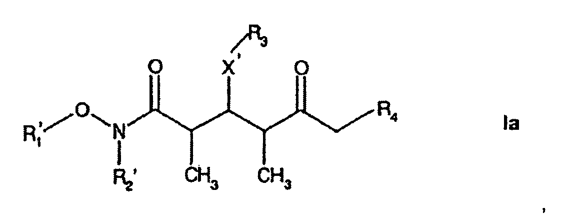 wobei
jedes R₁' und R₂' für (C₁-C₆)-Alkyl steht;
X' für O oder CH₂ steht; und
R₃ und R₄ wie in Anspruch 5 definiert sind.

7. Verbindung nach Anspruch 6 der Formel Ib: 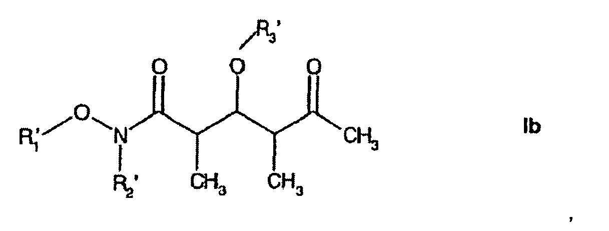 wobei
R₃' für (C₁-C₆)-Alkyl, C(O)(C₁-C₁₂)-Alkyl, Benzyl, C(O)O(C₁-C₁₂)-Alkyl oder eine säurelabile Hydroxylschutzgruppe steht; und
R₁' und R₂' wie in Anspruch 6 definiert sind.

8. Verbindung nach Anspruch 7 der Formel Ic: 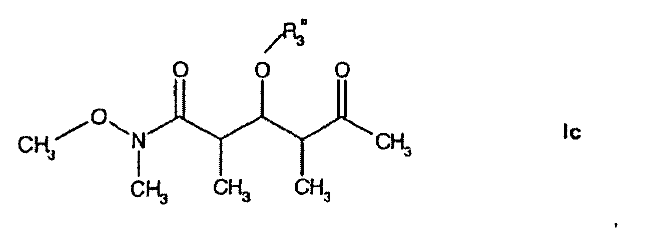 wobei R₃" für eine säurelabile Hydroxylschutzgruppe steht.

9. Verbindung nach Anspruch 8, die für (2R,3S,4R)-3-[[(1,1-Dimethylethyl)dimethylsilyl]oxy]-N-methoxy-N,2,4-trimethyl-5-oxo-hexanamid steht, das folgende Formel 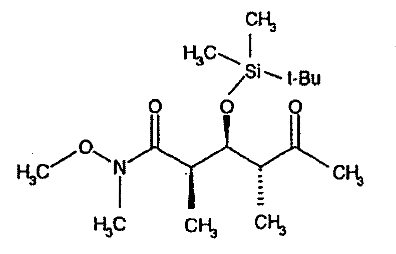 aufweist.

10. Verfahren zur Herstellung einer Verbindung der Formel I nach Anspruch 5, wobei das Verfahren in einer ersten Stufe eine Addition eines metallorganischen Methyl- oder Ethyl-Reagenzes zu einem Aldehyd der Formel VI 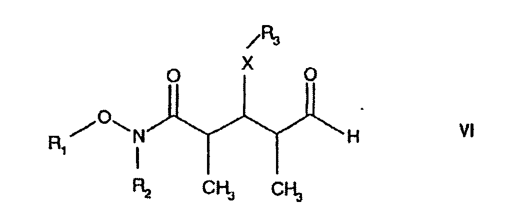 in Gegenwart eines polaren organischen Lösemittels umfasst, um einen Alkohol der Formel VII 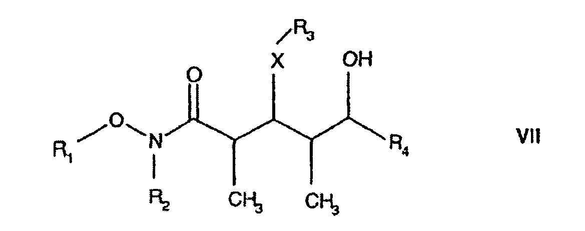 zu erhalten, und in einer zweiten Stufe eine Oxidation der in der ersten Stufe hergestellten Alkoholverbindung durch dessen Behandlung mit einem Oxidationsmittel und einer Base in einem polaren organischen Lösemittel, um die gewünschte Verbindung der Formel I zu erhalten, wobei R₁, R₂, R₃, R₄ und X wie in Anspruch 5 definiert sind.

11. Verfahren nach Anspruch 10, wobei die Additionsstufe in Gegenwart von Methylmagensiumbromid und Diethylether bei einer Temperatur von zwischen -78°C und 40°C ausgeführt wird.

12. Verfahren nach Anspruch 10, wobei die Oxidationsstufe in Gegenwart eines Schwefeltrioxid-Pyridin-Komplexes, Triethylamin und Dichlormethan bei einer Temperatur von zwischen -78°C und 40°C ausgeführt wird.

13. Verbindung der Formel III: 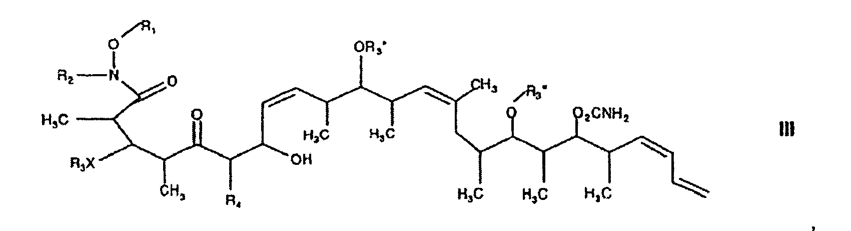 wobei
R₁ für (C₁-C₆)-Alkyl, Benzyl oder eine säurelabile Hydroxylschutzgruppe steht;
R₂ für (C₁-C₆)-Alkyl oder Benzyl steht;
R₃ für Wasserstoff, (C₁-C₆)-Alkyl, Benzyl, C(O)(C₁-C₁₂)-Alkyl, C(O)Ph, C(O)O(C₁-C₁₂)-Alkyl, C(O)OPh, C(O)NH(C₁-C₁₂)-Alkyl, C(O)NHPh oder eine säurelabile Hydroxylschutzgruppe steht;
R₄ für Wasserstoff oder Methyl steht;
X für O, NH, NCH₃, S oder CH₂ steht; und
jedes R₃" für eine säurelabile Hydroxylschutzgruppe steht.

14. Verbindung nach Anspruch 13, die für
(2R,3S,4R,7S,8Z,10S,11S,12S,13Z,16S,17R,18R,19S,20S,21E)-19-[(Aminocarbonyl)oxy]-3,11,17-tris[[(1,1-dimethylethyl)-dimethylsilyl]oxy]-7-hydroxy-N-methoxy-N,2,4,10,12,14,16,18,20-nonamethyl-5-oxo-8,13,21,23-tetracosatetraenamid steht, das die folgende Formel 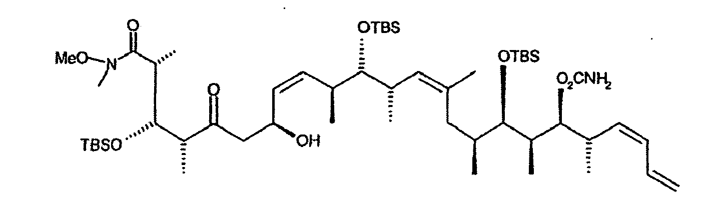 aufweist.

15. Verbindung der Formel IV: 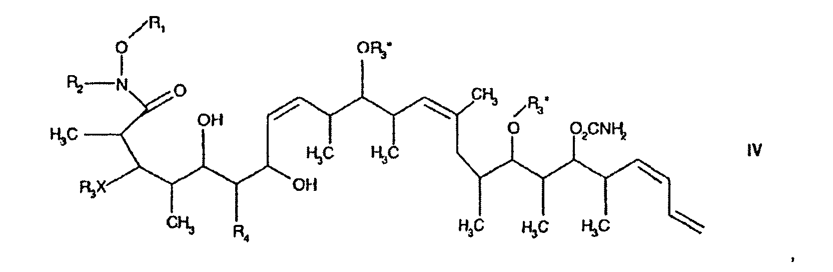 wobei
R₁ für (C₁-C₆)-Alkyl, Benzyl oder eine säurelabile Hydroxylschutzgruppe steht;
R₂ für (C₁-C₆)-Alkyl oder Benzyl steht;
R₃ für Wasserstoff, (C₁-C₆)-Alkyl, Benzyl, C(O)(C₁-C₁₂)-Alkyl, C(O)Ph, C(O)O(C₁-C₁₂)-Alkyl, C(O)OPh, C(O)NH(C₁-C₁₂)-Alkyl, C(O)NHPh oder eine säurelabile Hydroxylschutzgruppe steht;
R₄ für Wasserstoff oder Methyl steht;
X für O, NH, NCH₃, S oder CH₂ steht; und
jedes R₃" für eine säurelabile Hydroxylschutzgruppe steht.

16. Verbindung nach Anspruch 15, die für (2R,3S,4S,5S,7S,8Z,10S,11S,12S,13Z,16S,17R,18R,19S,20S,21E)-19-[(Aminocarbonyl)oxy]-3,11,17-tris[[(1,1-dimethylethyl)dimethylsilyl]oxy]-5,7-dihydroxy-N-methoxy-N,2,4,10,12,14,16,18,20-nonamethyl-8,13,21,23-tetracosatetraenamid steht, das die folgende Formel 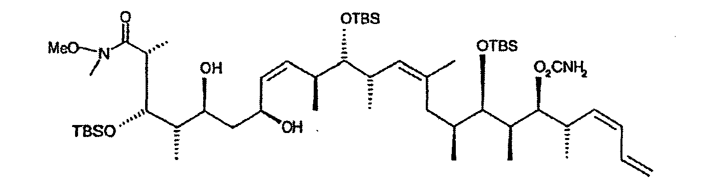 aufweist.

## Revendications

1. Procédé de préparation d'un composé de formule V 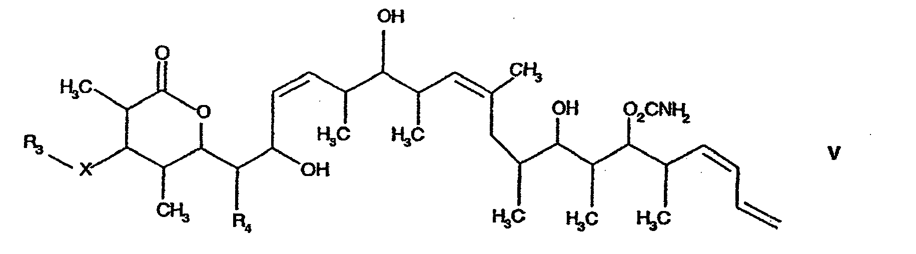 consistant, lors d'une première étape, à coupler un composé cétone de formule I 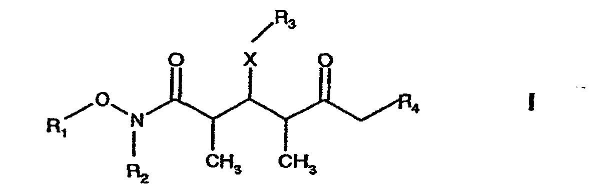 avec un composé aldéhyde de formule II 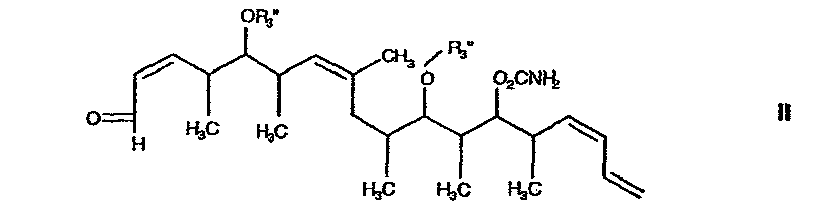 en présence d'un halogénure ou d'un triflate de dialkyle de bore, d'une base amine et d'un solvant organique polaire de façon à obtenir un β-hydroxycétone de formule III 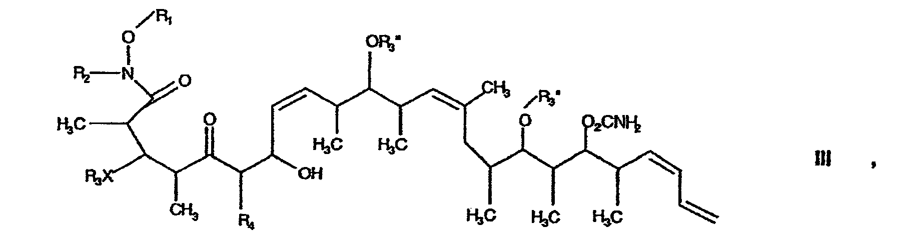 consistant, lors d'une seconde étape, à réduire le composé cétone obtenu lors de la première étape en le traitant avec un réactif borohydrure dans un solvant organique polaire et dans un solvant polaire de façon à obtenir un 1,3-diol de formule IV 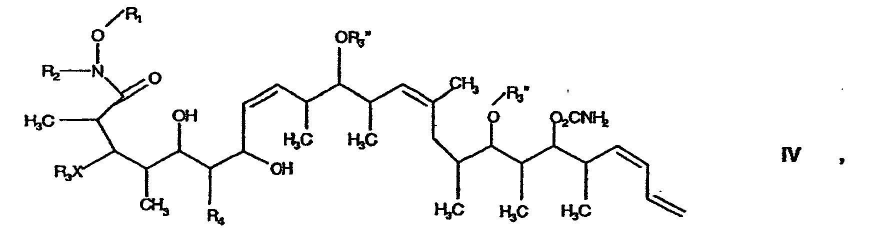 et, consistant, lors d'une troisième étape, à lactoniser et déprotéger les groupes protecteurs d'acide hydroxyle labile du 1,3-diole obtenu lors de la seconde étape en le traitant avec un halogénure d'hydrogène dissous dans un solvant polaire ou un mélange de solvants de façon à obtenir le composé souhaité de formule V, dans laquelle R₁ est (C₁₋₆)alkyle, benzyle ou un groupe protecteur d'acide hydroxyle labile ; R₂ est (C₁₋₆)alkyle ou benzyle ; R₃ est hydrogène, (C₁₋₆)alkyle, benzyle, C(O)(C₁₋₁₂)alkyle, C(O)Ph, C(O)O(C₁₋₁₂)alkyle, C(O)OPh, C(O)NH(C₁₋₁₂)alkyle, C(O)NHPh ou un groupe protecteur d'acide hydroxyle labile ; R₃" est un groupe protecteur d'acide hydroxyle labile ; R₄ est hydrogène ou méthyle ; et X est O, NH, NCH₃, S ou CH₂, à condition que lorsque X est O et que R₃ est un groupe protecteur d'acide hydroxyle labile, dans le composé de formule I, le groupement « -X-R₃ » dans le composé de formule V soit -OH.

2. Procédé selon la revendication 1, **caractérisé en ce que** l'étape de couplage est réalisée en présence de β-chlorodiisopinocamphénylborane, de triéthylamine et d'éther diéthylique à une température comprise dans la gamme allant de -78°C et -20°C.

3. Procédé selon la revendication 1, **caractérisé en ce que** l'étape de réduction est réalisée en présence de tétraméthylammonium, de triacétoxyborohydrure, d'acétonitrile et d'acide acétique à une température comprise dans la gamme allant de -40°C et -10°C.

4. Procédé selon la revendication 1, **caractérisé en ce que** la lactonisation et la déprotection des groupes protecteurs d'acide hydroxyle labile est réalisée en présence de chlorure d'hydrogène aqueux, de méthanol, d'acétonitrile et de tétrahydrofurane à une température comprise dans la gamme allant de -20°C et 40°C.

5. Composé de formule 1 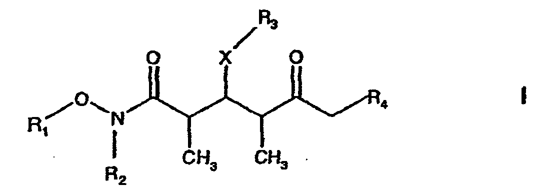 dans laquelle,
R₁ est (C₁₋₆)alkyle, benzyle ou un groupe protecteur d'acide hydroxyle labile ;
R₂ est (C₁₋₆)alkyle ou benzyle ;
R₃ est hydrogène, (C₁₋₆)alkyle, benzyle, C(O)(C₁₋₁₂)alkyle, C(O)Ph, C(O)O(C₁₋₁₂)alkyle, C(O)OPh, C(O)NH(C₁₋₁₂)alkyle, C(O)NHPh ou un groupe protecteur d'acide hydroxyle labile ;
R₄ est hydrogène ou méthyle ; et
X est O, NH, NCH₃, S ou CH₂.

6. Composé selon la revendication 5 de formule Ia : 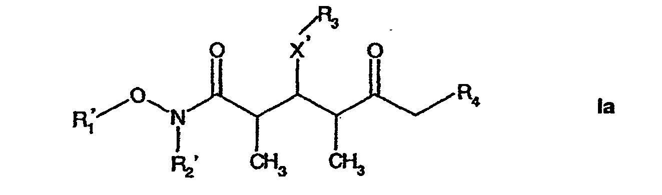 dans laquelle,
chacun des R₁' et R₂' est (C₁₋₆)alkyle ;
X' est O ou CH₂; et
R₃ et R₄ sont tels que définis à la revendication 5.

7. Composé selon la revendication 6 de formule Ib : 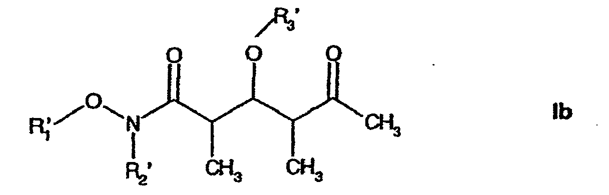 dans laquelle,
R₃' est (C₁₋₆)alkyle, C(O)(C₁₋₁₂)alkyle, benzyle, C(O)O(C₁₋₁₂)alkyle, ou un groupe protecteur d'acide hydroxyle labile ; et
R₁ et R₂ sont tels que définis à la revendication 6.

8. Composé selon la revendication 6 de formule Ic : 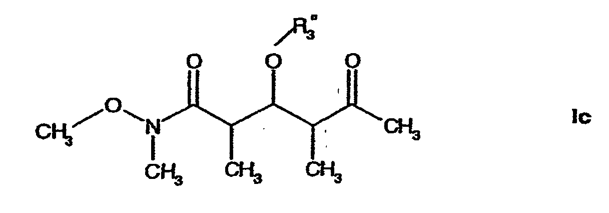 dans laquelle,
R₃" est un groupe protecteur d'acide hydroxyle labile.

9. Composé selon la revendication 8, dans laquelle (2*R*,3*S*,4*R*)-3-[[(1,1-diméthyléthyl)diméthylsilyl]oxy]-*N*-méthoxy-*N*,2,4-triméthyl-5-oxo-hexanamide de formule

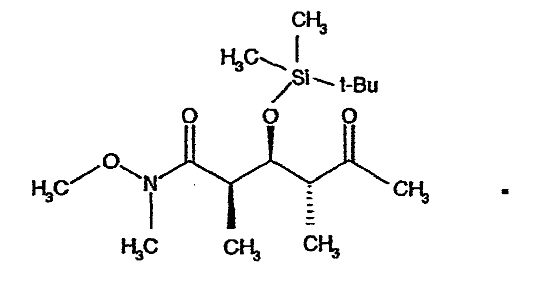

10. Procédé pour la préparation d'un composé de formule I selon la revendication 5 consistant, lors d'une première étape, à ajouter un réactif organométallique de méthyle ou d'éthyle à un aldéhyde de formule VI 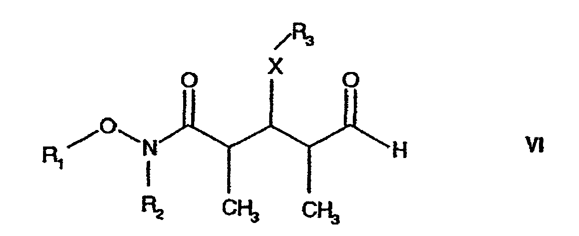 en présence d'un solvant organique polaire de façon à obtenir un alcool de formule VII 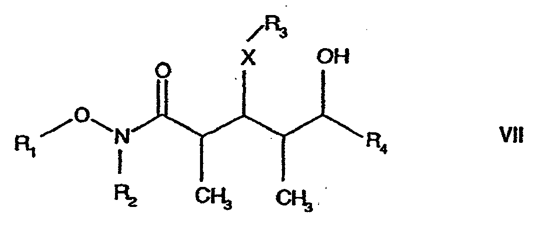 et, consistant, lors d'une seconde étape, à oxyder le composé alcool préparé lors de la première étape en le traitant avec un oxydant et une base dans un solvant organique polaire de façon à obtenir le composé souhaité de formule I dans laquelle R₁, R₂, R₃, R₄ et X sont tels que définis ci-dessus.

11. Procédé selon la revendication 10, **caractérisée en ce que** l'étape d'ajout est réalisée en présence de bromure de méthylmagnésium et d'éther diéthylique à une température comprise dans la gamme allant de -78°C à 40°C.

12. Procédé selon la revendication 10, **caractérisée en ce que** l'étape d'oxydation est réalisée en présence d'un complexe trioxyde-pyridine, de triéthylamine et de dichlorométhane à une température comprise dans la gamme allant de -78°C à 40°C.

13. Composé de formule III 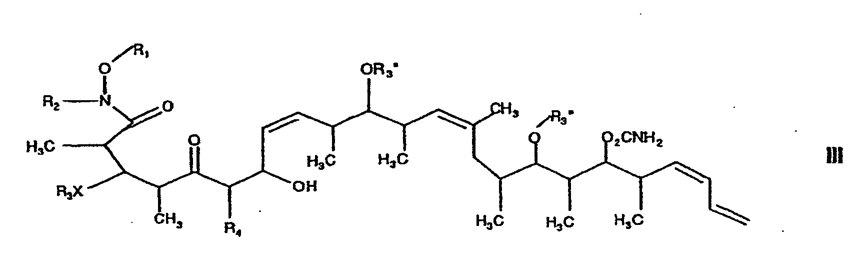 dans laquelle,
R₁ est (C₁₋₆)alkyle, benzyle ou un groupe protecteur d'acide hydroxyle labile ;
R₂ est (C₁₋₆)alkyle ou benzyle ;
R₃ est hydrogène, (C₁₋₆)alkyle, benzyle, C(O)(C₁₋₁₂)alkyle, C(O)Ph, C(O)O(C₁₋₁₂)alkyle, C(O)OPh, C(O)NH(C₁₋₁₂)alkyle, C(O)NHPh ou un groupe protecteur d'acide hydroxyle labile ;
R₄ est hydrogène ou méthyle ;
X est O, NH, NCH₃, S ou CH₂ ; et
chacun des R₃" est un groupe protecteur d'acide hydroxyle labile.

14. Composé selon la revendication 13 qui est (*2R,3S,4R,7S,8Z,10S, 11S,12S,13Z,16S,17R,18R,19S,20S,21E*)-19-[(aminocarbonyl)oxy]-3,11, 17-*tris*[[(1,1-diméthyléthyl)diméthylsilyl]oxy]-7-hydroxy-*N*-méthoxy-*N*,2, 4,10,12,14,16,18,20-nonaméthyl-5-oxo-8,13,21,23-tétracosatétraenamide de formule

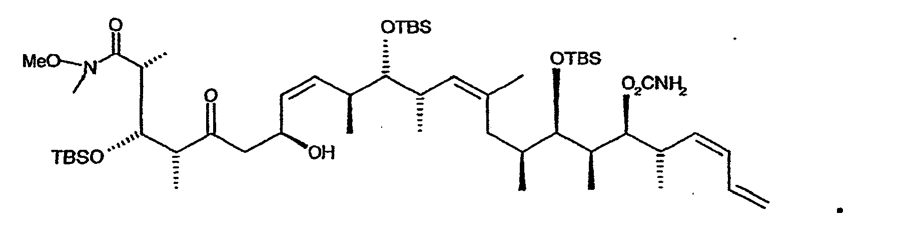

15. Composé de formule IV : 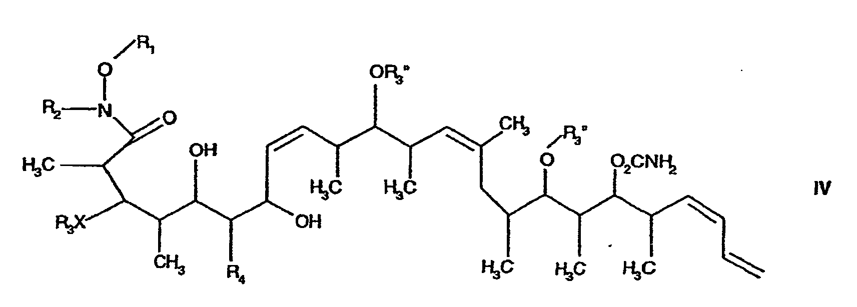 dans laquelle,
R₁ est (C₁₋₆)alkyle, benzyle ou un groupe protecteur d'acide hydroxyle labile ;
R₂ est (C₁₋₆)alkyle ou benzyle ;
R₃ est hydrogène, (C₁₋₆)alkyle, benzyle, C(O)(C₁₋₁₂)alkyle, C(O)Ph, C(O)O(C₁₋₁₂)alkyle, C(O)OPh, C(O)NH(C₁₋₁₂)alkyle, C(O)NHPh ou un groupe protecteur d'acide hydroxyle labile ;
R₄ est hydrogène ou méthyle ;
X est O, NH, NCH₃, S ou CH₂ ; et
chacun des R₃" est un groupe protecteur d'acide hydroxyle labile.

16. Composé selon la revendication 15 qui est (2R, 3*S*, *4S, 5S, 7S, 8Z, 10S, 11S,125,13Z,16S,17R,18R,19S,20S,21E*)-19-[(aminocarbonyl)oxy]-3,11, 17*-tris*[[(1,1-diméthyléthyl)diméthylsilyl]oxy]-5,7-dihydroxy-*N*-méthoxy-*N*,2,4,10,12,14,16,18,20-nonaméthyl-8,13,21,23-tétracosatétra-enamide de formule
